# EUROPÄISCHE PATENTSCHRIFT

(11) **EP 1 405 615 B1**
(45) Veröffentlichungstag und Bekanntmachung des Hinweises auf die Patenterteilung: **18.10.2006**
(21) Anmeldenummer: 03017959.2
(22) Anmeldetag: 06.08.2003
(51) Int. Cl.: A61F 2/44

(54) **Bandscheibenprothese**
Spinal disc prosthesis
Prothèse de disque intervertébral

(30) Priorität: 12.09.2002 DE 10242329
(43) Veröffentlichungstag der Anmeldung: 07.04.2004
(60) Teilanmeldung: 05021536.7
(73) Patentinhaber: BIEDERMANN MOTECH GmbH, 78054 Villingen-Schwenningen (DE)
(72) Erfinder: Biedermann, Lutz, 78048 VS-Villingen (DE); Harms, Jürgen, 76227 Karlsruhe (DE)
(74) Vertreter: Hofer, Dorothea

(56) Entgegenhaltungen:
- WO-A-00/35384
- WO-A-01/68003
- DE-A- 2 263 842
- DE-A- 4 208 116
- US-A- 5 314 477
- US-A1- 2003 045 939

## Beschreibung

Die Erfindung betrifft eine Bandscheibenprothese nach dem Oberbegriff des Patentanspruches 1.

Aus der DE 42 08 116 C ist eine derartige Bandscheibenprothese bekannt. Aus der EP 0 471 821 B ist ferner eine Bandscheibenprothese bekannt, die einen Kern aufweist, der einseitig sphärisch ausgebildet ist.

Aus der US 5,314,477 ist eine Bandscheibenprothese mit einer Grundplatte und einer dieser gegenüberliegenden Deckplatte und einem dazwischenliegenden Kern bekannt, wobei eine der Platten auf der dem Kern zugewandten Seite eine erste konkave Kontaktfläche und der Kern wenigstens eine angrenzende erste konvexe Kontaktfläche aufweist. Die einander zugewandten Flächen der anderen Platte und des Kerns sind eben. Zwischen diesen Flächen befindet sich eine flexible Unterlegscheibe.

Aus der DE-OS 2 263 842 ist eine Bandscheibenprothese bekannt, die als oben und unten zumindest angenähert linsenförmig gewölbte Stirnflächen aufweisende Scheibe ausgebildet ist, wobei eine elastische Zwischenschicht vorgesehen ist, die sich in einer Normalebene zur Prothesenachse erstreckt.

Aufgabe der Erfindung ist es, eine Bandscheibenprothese der eingangs beschriebenen Art zu schaffen, die gegenüber dem Stand der Technik verbessert ist.

Diese Aufgabe wird durch eine Bandscheibenprothese nach Patentanspruch 1 gelöst.

Bei der Bandscheibenprothese nach Patentanspruch 1 ist dabei insbesondere eine Dämpfung im Endbereich der Kippbewegung vorgesehen.

Ferner wird eine Vertikaldämpfung verbessert, sowie eine axiale Rotation begrenzt.

Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren. Von den Figuren zeigen:
- Fig. 1: eine perspektivische Seitenansicht einer ersten Ausführungsform;
- Fig. 2: einen Schnitt durch die in Fig. 1 gezeigte Ausführungsform;
- Fig. 3: eine perspektivische Seitenansicht eines weiteren Beispiels;
- Fig. 4: einen Schnitt durch das in Fig. 3 gezeigte Beispiel;
- Fig. 5: einen Schnitt durch ein drittes Beispiel;
- Fig. 6: einen Schnitt durch ein viertes Beispiel;
- Fig. 7: eine perspektivische Ansicht des in Fig. 6 gezeigten Beispiels; und
- Fig. 8-10: Draufsichten eines Details.

Wie aus den Figuren ersichtlich ist, weist die erste Ausführungsform der Bandscheibenprothese sowie alle genannten Beispiele eine Grundplatte 1, eine dieser gegenüberliegende Deckplatte 2 und einen dazwischenliegenden Kern 3 auf.

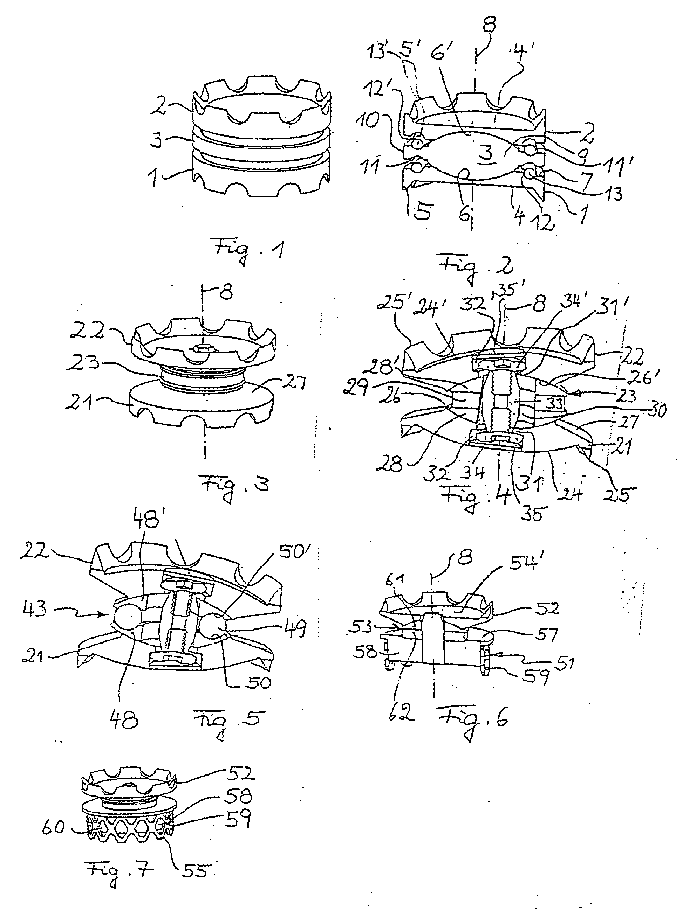

Bei dem in den Figuren 1 und 2 gezeigten ersten Ausführungsbeispiel weist die Grundplatte 1 auf der dem Kern abgewandten Außenseite eine ebene Außenfläche 4 auf. An ihrem äußeren Rand weist die Grundplatte nach außen sich zu der Außenfläche vertikal erstreckende Zacken 5 auf, die zum Eingreifen in eine benachbarte Wandung eines Wirbelkörpers dienen. Auf der der Außenfläche 4 gegenüberliegenden Innenseite weist die Grundplatte eine konkave Ausnehmung 6 auf, die vorzugsweise als sphärisches Segment ausgebildet ist. Angrenzend an die konkave Ausnehmung und um diese herumlaufend erstreckt sich eine zu der Außenfläche 4 parallele Randzone 7.

Wie aus Fig. 2 ersichtlich ist, ist die Deckplatte 2 identisch zur Grundplatte 1 ausgebildet und lediglich spiegelsymmetrisch angeordnet, so daß die konkave Ausnehmung 6' der Deckplatte 2 der konkaven Ausnehmung 6 der Grundplatte 1 zugewandt ist.

Zwischen Grundplatte 1 und Deckplatte 2 liegt der Kern 3. Dieser weist einen zur Symmetrieachse 8 symmetrisch angeordneten Zentralteil 9 auf, der die Form einer bikonvexen Linse aufweist und dessen jeweils konvexe Außenflächen die gleiche Krümmung und insbesondere sphärische Krümmung aufweisen wie die damit zusammenwirkenden konkaven Ausnehmungen 6 bzw. 6' der Grundplatte und Deckplatte.

Wie aus Fig. 2 weiter ersichtlich ist, weist auch der Kern 3 eine Randzone 10 auf, deren Außendurchmesser gleich dem Durchmesser von Grund- und Deckplatte ist. Die Randzone ist vorzugsweise so ausgebildet, daß die beiden der Grundplatte und der Deckplatte zugewandten Flächen zueinander und zu der Symmetrieebene des Kerns parallel ausgebildet sind.

Wie weiter aus Fig. 2 ersichtlich ist, weist die Randzone 10 auf der der Grundplatte 1 zugewandten Unterseite benachbart zu dem Zentralteil 9 eine ringförmige Ausnehmung 11 auf. Diese weist in dem gezeigten Ausführungsbeispiel einen kreissegmentförmigen Querschnitt auf. Die dem gegenüberliegende Fläche der Randzone 7 der Grundplatte weist eine den gleichen Durchmesser aufweisende ringförmige Ausnehmung 12 auf, die ebenfalls einen kreissegmentförmigen Querschnitt besitzt.

Wie aus Fig. 2 ersichtlich ist, ist die der Deckplatte 2 zugewandte Oberfläche der Randzone 10 symmetrisch zu der der Grundplatte zugewandten Seite ausgebildet und weist eine entsprechende ringförmige Ausnehmung 11' auf. Die dem Kern zugewandte Seite der Deckplatte 2 weist wie die Grundplatte 1 ebenfalls eine der ringförmigen Ausnehmung 11' gegenüberliegende ringförmige Ausnehmung 12' auf, die in ihren Abmessungen der ringförmigen Ausnehmung 11' entspricht.

Wie aus Fig. 2 ferner ersichtlich ist, sind in den jeweiligen Paaren 11, 12 bzw. 11', 12' der ringförmigen Ausnehmungen Ringe 13 bzw. 13' angeordnet.

Die Grundplatte 1 und die Deckplatte 2 sind aus einem biokompatiblen Material, insbesondere Stahl oder Titan hergestellt. Der Kern 3 ist nach einer ersten Ausführungsform aus einem körperverträglichen hochmolekularen Polyethylenkunststoff geformt. Die beiden Ringe sind aus einem körperverträglichen elastischen Kunststoff, beispielsweise Medical Grade Silikongummi gebildet.

Bei der Anwendung wird die so ausgebildete Bandscheibenprothese nach Entfernen der geschädigten Bandscheibe zwischen zwei Wirbelkörper eingesetzt und greift mit den Zacken 5, 5' in die benachbarten Wirbelkörperwandungen ein, so daß die Platten selbst drehfest gehalten werden. Die Ringe 13, 13' bewirken eine Abfederung der Bandscheibenprothese gegen zu starkes Verkippen und bremsen gleichzeitig ein zu starkes Verdrehen um die Mittenachse 8.

Der Außendurchmesser von Grund- und Deckplatte ist so gewählt, daß er wenig kleiner ist als der kleinste Durchmesser der benachbarten Wirbelkörperendplattenfläche.

Die in den Figuren 3 bis 6 gezeigten Beispiele dienen nur einer technischen Erläuterung und sind nicht Bestandteil der beanspruchten Erfindung.

Auch die in den Figuren 3, 4 und 5 gezeigten Ausführungsbeispiele sind jeweils um die sich senkrecht zur Symmetrieachse 8 erstreckende Mittenebene spiegelsymmetrisch ausgebildet.

Das in den Figuren 3 und 4 gezeigte zweite Beispiel weist wiederum eine Grundplatte 21, eine Deckplatte 22 und dazwischen einen Kern 23 auf.

Die Grundplatte 21 weist wiederum nach außen hervorstehende Zacken 25 auf. Die Außenfläche 24 ist, wie am besten aus Fig. 4 ersichtlich ist, als konvexe kugelsegmentförmige Oberfläche ausgebildet, wobei die Krümmung der Oberfläche so gewählt ist, daß sie im wesentlichen einer typischen konkaven Krümmung einer damit in Kontakt zu bringenden Wirbelkörperendplattenfläche entspricht. Symmetrisch zur Symmetrieachse 8 weist die dem Kern zugewandte Oberfläche eine der konkaven Ausnehmung 6 entsprechende konkave Ausnehmung 26 auf. Es ist eine erste Randzone 27 vorgesehen, die anders als beim ersten Ausführungsbeispiel aber nicht eben, sondern zur Außenseite der Grundplatte hin kegelstumpfförmig abfallend ausgebildet ist.

Wie aus Fig. 4 ersichtlich ist, ist die Deckplatte 22 identisch zur Grundplatte ausgebildet und zu einer sich senkrecht zur Symmetrieachse 8 erstreckenden Mittenebene spiegelsymmetrisch angeordnet.

Der Kern 23 ist dreiteilig ausgebildet und besteht aus zwei mit ihren Planflächen einander zugewandten plan-konvexen Linsenkörpern 28, zwischen denen eine plan-parallele Platte 29 angeordnet ist. Die Linsenkörper 28, 28` und die Platte 29 haben im wesentlichen den gleichen Durchmesser. Die Krümmung der konvexen Flächen der Linsenkörper entspricht der Krümmung der damit zusammenwirkenden konkaven Ausnehmungen 26, 26'.

Wie am besten aus Fig. 4 ersichtlich ist, weist der Kern 23 eine sich senkrecht zu seiner Symmetrieebene erstreckende und durch seinen Mittelpunkt gehende Bohrung 30 auf. An den entsprechenden Stellen weisen Grundplatte und Deckplatte sich entlang ihrer Symmetrieachsen erstreckende durchgehende Ausnehmungen 31, 31' auf. Auf den jeweiligen den Außenflächen 24, 24' zugewandten Seiten sind diese durch Senkbohrungen 32, 32' in ihrem Durchmesser erweitert. In der Bohrung 30 ist eine vorzugsweise aus einem körperverträglichen Kunststoff oder aus Metall gefertigte Verbindungshülse 33 vorgesehen, deren Durchmesser kleiner ist als der Durchmesser der Bohrung 30 und deren Länge größer als die Länge der Bohrung 30 ist, so daß die Verbindungshülse mit dem jeweiligen freien Ende in die Ausnehmung der benachbarten Platte eingreift. Wie aus Fig. 4 ersichtlich ist, ist die Hülse zu ihren Enden hin jeweils verjüngt ausgebildet. Von beiden Seiten ist jeweils durch die Ausnehmungen 31 geführt eine Schraube 34, 34' in die Verbindungshülse 33 eingeschraubt, wobei der Kopf der Schraube stets in der Senkbohrung anliegt. Die Senkbohrung ist ein wenig größer als der jeweilige Kopf. Die Schrauben werden so weit angezogen, daß Grund- und Deckplatte und Kern so miteinander verbunden sind, daß die aneinandergrenzenden Flächen ohne Spiel, aber zueinander beweglich gehalten sind.

Wie aus Fig. 4 ersichtlich ist, ist die Tiefe der Senkbohrungen 32, 32' etwas größer als die Dicke der Köpfe der Schrauben 34, 34'. Die Senkbohrungen sind an ihrem äußeren Ende jeweils durch Abdeckplatten 35 nach außen hin abgedeckt. Der Unterschied zwischen der Tiefe der Senkbohrungen 32, 32' und der Dicke der Köpfe der Schrauben 34, 34' ist so gewählt, daß die Köpfe beim federnden Zusammendrücken der Bandscheibenprothese gerade noch nicht an die Abdeckplatten 35 stoßen.

Die Grund- und Deckplatten sind vorzugsweise aus dem gleichen Material ausgebildet wie bei der ersten Ausführungsform. Die Linsenkörper 28, 28' haben vorzugswiese die gleiche Materialausbildung wie die Grund- und Deckplatte. Die Platte 29 ist aus einem körperverträglichen elastischen Kunststoff, vorzugsweise einem Medical Grade Silikongummi gebildet. Auf diese Weise übernehmen die Linsenkörper zusammen mit den Grund- und Deckplatten die Kippbewegung, während die Platte 29 für die Elastizität und damit die Abfederung sorgt.

Das in Fig. 5 gezeigte Beispiel unterscheidet sich von dem in Fig. 3 und 4 gezeigten Beispiel nur durch die Ausbildung des Kernes. Alle übrigen Teile stimmen mit dem zuvor beschriebenen Beispiel überein.

Der Kern 43 weist wiederum zwei äußere plan-konvexe Linsenkörper 48, 48' auf, die mit ihren konvexen Flächen in gleicher Weise wie vorher beschrieben mit den Grund- und Deckplatten zusammenwirken. Auch die zentrale Bohrung und die Befestigung mittels der Verbindungshülse und den Schrauben stimmt identisch überein. Anders als bei dem vorherigen Ausführungsbeispiel ist anstelle der plan-parallelen Platte 29 ein elastischer Ring 49 vorgesehen. Zur Aufnahme und Führung des Ringes 49 weisen die einander zugewandten planen Flächen der Linsenkörper 48, 48' im Querschnitt kreisseigmentförmige ringförmige Ausnehmungen 50, 50' auf, in denen der Ring 49 gehalten ist.

Die Materialien und die Funktionsweise und das Einbauen entsprechen dem vorher beschriebenen Beispiel. Der Ring übernimmt die Funktion der Platte.

Bei dem in Fig. 6 gezeigten weiteren Beispiel stimmt die Deckplatte mit der in Fig. 4 beschriebenen Deckplatte überein. In der Fig. 6 ist die Außenfläche 54 eben dargestellt, sie kann aber auch wie in dem in Fig. 4 gezeigten Beispiel der Fläche 24' entsprechend konvex ausgebildet sein.

Anders ausgebildet dagegen ist die Grundplatte 51. Diese ist als Zylinderelement ausgebildet, welches auf seiner der Deckplatte zugewandten Seite eine ebene Fläche 57 mit einem Durchmesser, der gleich dem Durchmesser der Deckplatte ist, aufweist. Auf seiner der Deckplatte abgewandten Seite schließt sich ein zylindrischer Abschnitt 58 an, dessen Durchmesser ein wenig kleiner ist, so daß der darüberliegende Abschnitt mit größerem Durchmesser einen Anschlag bildet. Der Abschnitt 58 dient zum Aufnehmen eines Zylindermantels 59, der am besten in der perspektivischen Darstellung in Fig. 7 ersichtlich ist. Der Zylindermantel wird im Paßsitz auf den Abschnitt 58 aufgesetzt und weist an seinem freien Ende mit dem benachbarten Wirbelkörper in Eingriff bringbare Zacken 55 auf. Ferner weist der Zylindermantel Ausnehmungen 60 auf, die die Einwachsmöglichkeit wesentlich verbessern.

Der Kern 53 weist auf seiner der Deckplatt 52 zugewandten Seite einen plan-konvexen Linsenkörper 61 auf, der dem Linsenkörper 28' in Form und Material entspricht. Auf der diesem Linsenkörper abgewandten Seite ist zwischen seiner planen Oberfläche und der ebenen Oberfläche 57 der Grundplatte 51 eine plan-parallele Platte 62 vorgesehen. Die in Fig.6 lediglich schematisch dargestellte Verbindung zwischen Grundplatte 51 und Deckplatte 52 mit dem Kern 53 dazwischen ist in der gleichen Weise wie vorhergehend beschrieben ausgebildet. Bei diesem Beispiel erfolgt die Bewegung über das Gleitpaar Linsenkörper 61 und Deckplatte 52. Die Dämpfung wird von der Platte 62 übernommen.

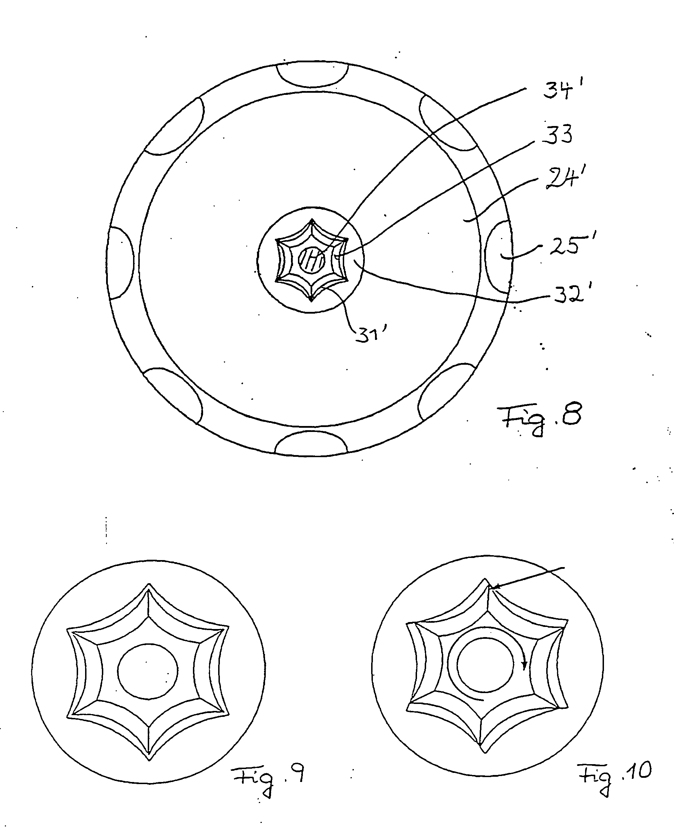

In Fig. 8 ist eine Draufsicht auf eine Deckplatte der in den Fig. 3 bis 7 beschriebenen Beispielen gezeigt, wobei die Abdeckplatte 35' und der Kopf der Schraube 34' weggelassen sind.

Aus Fig. 8 ist zu ersehen, daß die jeweilige Hülse 33 an ihren jeweils verjüngt abgeschrägten Enden sechskantig ausgebildet ist, wobei die Flächen zwischen den sechs Ecken jeweils hohlkehlenartig ausgebildet sind. Die diesen sechskantigen Abschnitt aufnehmende jeweilige Ausnehmung 31' ist ebenfalls sechskantig ausgebildet, wobei der jeweilige Durchmesser durch zwei gegenüberliegende Ecken jeweils um ein vorbestimmtes Maß wenig größer als der entsprechende Durchmesser der Verbindungshülse an dieser Stelle ist. Die Flächen zwischen jeweils zwei Ecken sind zur Mitte der Ausnehmung hin bauchig ausgebildet, wobei der Radius der bauchigen Krümmung jeweils um ein vorbestimmtes Maß wenig größer als der Radius der Hohlkehlen ist.

Wie in Fig. 9 und Fig. 10 gezeigt ist, kann somit eine Drehung um ein durch die Größenunterschiede vorbestimmtes Maß zwischen Hülse und Deckplatte bzw. Hülse und Grundplatte erfolgen. Damit wird eine Begrenzung der Drehung auf einen vorbestimmten Winkel erreicht.

Bei allen gezeigten Ausführungsformen und Beispielen können die Außenflächen von Grund- und Deckfläche rauh ausgebildet sein, um eine Verbesserung des Einwachsens zu erreichen.

Bei allen oben beschriebenen Ausführungsbeispielen können die aneinandergrenzenden und eine Relativbewegung zueinander ausführenden Flächen mit entsprechendem Material als Gleitpaarung beschichtet sein. Dafür kommen insbesondere Keramikschichten oder auch Polyethylenbeschichtungen oder auch entsprechende Metallegierungen in Frage.

Bei den oben beschriebenen Ausführungsformen und Beispielen sind jeweils aneinandergrenzende und zusammenwirkende konkave und konvexe sphärische Flächen beschrieben. Dabei hat jeweils der Kern die konvexen Flächen und die Deckplatte und die Grundplatte haben zugehörige konkave sphärische Flächen. Nach einer abgewandelten Ausführungsform können die Flächenformen jeweils umgekehrt sein. Das heißt, der Kern kann als bikonkaver Linsenkörper oder als plan-konkaver Linsenkörper ausgebildet sein und die zugehörige Kontaktfläche von Grundplatte und Deckplatte ist dann entsprechend zu der konkaven sphärischen Fläche sphärisch konvex ausgebildet.

## Patentansprüche

1. Bandscheibenprothese mit einer Grundplatte (1) und einer dieser gegenüberliegenden Deckplatte (2) und einem dazwischenliegenden Kern (3),
wobei wenigstens eine der Platten auf der dem Kern zugewandten Seite eine erste konkave Kontaktfläche (6) und der Kern wenigstens eine angrenzende erste konvexe Kontaktfläche aufweisen oder,
wobei die Platten auf den dem Kern zugewandten Seiten konvexe erste Kontaktflächen aufweisen und der Kern angrenzende konkave zweite Kontaktflächen aufweist,
**dadurch gekennzeichnet, daß** um eine der Kontaktflächen herum eine Rille (11, 11', 12, 12') vorgesehen ist, in der ein mit der gegenüberliegenden Kontaktfläche in Kontakt befindlicher elastischer erster Ring (13, 13') eingebettet ist.

2. Bandscheibenprothese nach Anspruch 1, **dadurch gekennzeichnet, daß** auch um die gegenüberliegende Kontaktfläche herum eine Rille vorgesehen ist, in die der erste Ring eingreift.

3. Bandscheibenprothese nach Anspruch 1 oder 2, **dadurch gekennzeichnet, daß** auch um eine der zweiten Kontaktflächen herum eine Rille vorgesehen ist, in der ein mit der gegenüberliegenden Kontaktfläche in Kontakt befindlicher elastischer zweiter Ring eingebettet ist.

4. Bandscheibenprothese nach Anspruch 3, **dadurch gekennzeichnet, daß** auch um die mit dem zweiten Ring in Kontakt befindliche gegenüberliegende Kontaktfläche herum eine entsprechende Rille vorgesehen ist.

## Claims

1. Intervertebral disc prosthesis with a base plate (1) and a top plate (2) opposite thereto and an intervening core (3),
wherein at least one of the plates, on the side facing the core, has a first concave contact face (6) and the core has at least one adjacent first convex contact face or
wherein the plates, on the sides facing the core, comprise convex first contact faces and the core comprises adjacent concave second contact faces,
**characterized in that** encircling one of the contact faces a groove (11, 11', 12, 12') is provided in which an elastic first ring (13, 13') being in contact with the opposite contact face is embedded.

2. Intervertebral disc prosthesis according to claim 1, **characterized in that**, also encircling the opposite contact face, a groove is provided in which the first ring engages.

3. Intervertebral disc prosthesis according to claim 1 or 2, **characterized in that**, also encircling one of the second contact faces, a groove is provided in which an elastic second ring being in contact with the opposite contact face is embedded.

4. Intervertebral disc prosthesis according to claim 3, **characterized in that**, also encircling the opposite contact face being in contact with the second ring, a corresponding groove is provided.

## Revendications

1. Prothèse de disque intervertébral avec une plaque de base (1) et une plaque de couverture (2) se trouvant à l'opposé de cette dernière, et un noyau (3) se trouvant entre les deux,
dans laquelle au moins une des plaques comporte sur la face tournée vers le noyau une première surface de contact (6) concave et le noyau au moins une première surface de contact adjacente convexe ou
dans laquelle les plaques sur les faces tournées vers le noyau comportent des premières surfaces de contact convexes et le noyau comporte des deuxièmes surfaces de contact adjacentes concaves,
**caractérisée en ce qu'**autour d'une des surfaces de contact est prévue une rainure (11, 11', 12, 12'), dans laquelle est logé un premier anneau élastique (13, 13') se trouvant en contact avec les surfaces de contact se faisant face.

2. Prothèse de disque intervertébral selon la revendication 1, **caractérisée en ce qu'**une rainure, dans laquelle le premier anneau vient en prise, est aussi prévue autour de la surface de contact opposée.

3. Prothèse de disque intervertébral selon la revendication 1 ou 2, **caractérisée en ce qu'**une rainure est aussi prévue autour d'une des deuxièmes surfaces de contact, rainure dans laquelle est logé un deuxième anneau élastique se trouvant en contact avec la surface de contact opposée.

4. Prothèse de disque intervertébral selon la revendication 3, **caractérisée en ce qu'**une rainure correspondante est aussi prévue autour de la surface de contact opposée se trouvant en contact avec le deuxième anneau.
